# DEMANDE DE BREVET EUROPEEN

(11) **EP 0 522 929 A1**
(43) Date de publication de la demande: **13.01.1993**
(21) Numéro de dépôt: 92401874.0
(22) Date de dépôt: 01.07.1992
(51) Int. Cl.: C08B 31/00, A23L 1/00, A23K 1/00, C12P 19/14, C12P 7/06, C13K 1/06

(54) **Procédé de production en continu d'amidon partiellement hydrolysé, produit obtenu par ce procédé et ses applications**

(30) Priorité: 03.07.1991 FR 9108286
(71) Demandeur: BIODYNE BOURGOGNE, F-89000 Auxerre (FR)
(72) Inventeur: Ramirez, Alain, F-86000 Poitiers (FR); Ghozlan, Dominique, F-30150 Sauveterre (FR)
(74) Mandataire: Serin, Jean-Pierre

(57) **Abrégé**

Procédé de production en continu d'amidon partiellement hydrolysé, par hydrolyse à froid et fermentation simultanées d'un substrat amylacé brut broyé, dans lequel on réalise de manière connue en soi une hydrolyse du substrat avec une préparation enzymatique libérant des sucres, et dans laquelle on ajoute des levures. Dans ce procédé,
- on soutire le jus obtenu après hydrolyse pendant une durée allant de 20 à 60 heures, lequel contient les produits solubilisés, et les produits non solubilisés.
- de ce jus on sépare tout ce qui n'est pas solubilisé par l'hydrolyse (notamment les fibres, les enzymes et amidons partiellement hydrolysés) que l'on recycle,
- on extrait avant recyclage et en tout ou partie l'amidon partiellement hydrolysé et on le stabilise.

## Description

La présente invention se rapporte à un procédé de production en continu d'amidon partiellement hydrolysé, par hydrolyse à froid et fermentation simultanées d'un substrat amylacé brut broyé, au produit obtenu et aux applications de ce produit.

Le brevet français 86.18380 décrit un procédé de production d'éthanol en continu, par hydrolyse et fermentation simultanées et à froid d'un substrat amylacé broyé. Par l'expression "à froid" on entend une température inférieure au point de gélification du substrat.

Selon ce procédé on introduit en continu dans un récipient contenant des ferments une certaine quantité de farine, de l'eau, des enzymes; on maintient dans le récipient une température basse; après un temps de séjour du milieu de fermentation dans le récipient compris entre 10 et 40 heures, on soutire les jus obtenus. Dans un séparateur on extrait du jus la fraction insoluble qui est recyclée dans le récipient; les jus restants ainsi clarifiés sont distillés, les vinasses étant recyclées ou non dans le récipient. La fraction insoluble comporte de la matière amylacée incomplètement hydrolysée et des constituants non transformables (levures, sons,...).

Cette fraction insoluble étant continuellement recyclée, il est nécessaire d'en éliminer une partie pour conserver le taux de matières sèches constant dans le récipient. L'élimination a lieu par soutirage après le séparateur, sur la conduite de retour au récipient. De même pour les vinasses on prévoit une purge en sortie de colonne de distillation. On assure ainsi l'équilibre entre les quantités entrantes et sortantes.

Ce procédé donne satisfaction car il permet d'obtenir dans la fraction de jus clarifiés une concentration en alcool comprise entre 48 et 80 g/l.

Les produits éliminés participent à l'élaboration des drêches. Dans le domaine agro-alimentaire, les drêches sont très généralement les résidus des céréales après soutirage des vins. Dans le procédé ci-dessus les drêches sont obtenues de la manière suivante, connue en soi: les vinasses sont constituées d'eau, de protéines précipitées et non précipitées, et de sel minéraux. Par centrifugation on sépare les protéines précipitées (solides) que l'on sèche, de la fraction solubilisée appelée vinasse clarifiée. Une partie des vinasses clarifiées est recyclée dans le récipient, l'autre est concentrée et mélangée à la fraction solide séchée; le mélange est communément désigné: "drêches solubles".

En ce qui concerne la partie insoluble de la fraction des jus qui est éliminée après le séparateur, celle-ci comporte comme on l'a vu de la matière amylacée incomplètement hydrolysée. Cette matière, qui n'est ni un sucre, et qui n'est plus un amidon natif, est considérée comme étant inutilisable: c'est un déchet provenant de la décomposition inachevée de la matière amylacée; et en outre elle est instable car sa transformation se poursuit encore lentement même après le soutirage. On incorpore aux drêches issues des vinasses cette matière amylacée afin de ne pas la perdre totalement, l'ensemble étant ensuite séché comme d'habitude.

Ce procédé présente l'inconvénient de ne pas transformer la totalité de la matière amylacée en alcool et de perdre la partie non transformée. Et cette perte est assez importante du point de vue économique car si les drêches solubles constituent en elles-mêmes un produit commercialisable leur prix est bien moindre que celui du produit amylacé. La présente invention a pour objectif de résoudre cet inconvénient.

L'invention a pour objet un procédé de production en continu d'amidon partiellement hydrolysé, par hydrolyse à froid et fermentation simultanées d'un substrat amylacé brut broyé, dans lequel on réalise de manière connue en soi une hydrolyse du substrat amylacé avec une préparation enzymatique libérant des sucres, et dans laquelle on ajoute des levures, caractérisé en ce que :
- on soutire le jus obtenu après hydrolyse et fermentation pendant une durée allant de au moins 20 heures à 60 heures, lequel contient notamment les produits solubilisés, et des produits non solubilisés,
- de ce jus on sépare tout ce qui n'est pas solubilisé par l'hydrolyse (notamment les fibres, les levures, l'enzyme et l'amidon partiellement hydrolysé) que l'on recycle,
- avant recyclage on extrait en tout ou partie cette fraction non solubilisée contenant l'amidon partiellement hydrolysé et on la stabilise par tout moyen connu, notamment par séchage, congélation, atomisation ou ensilage.

La durée d'hydrolyse est de préférence comprise entre 35 et 60 heures.

Comme on le voit le procédé consiste à interrompre un processus de transformation par hydrolyse et fermentation simultanée, à extraire l'hydrolysat inachevé c'est-à-dire le produit intermédiaire et à arrêter l'hydrolyse.

On s'est aperçu en effet que l'amidon ainsi partiellement dégradé présentait une apparence très différente de l'amidon cru ou natif: alors que le grain d'amidon cru est lisse, le grain partiellement hydrolysé présente une multitude de creux et/ou de petites cavernes résultant d'une décomposition en cours et que le milieu dans lequel ces grains dégradés se trouvent est biologiquement sain, les germes pathogènes étant dans l'impossibilité de se multiplier de manière importante, en raison de la présence des levures, leurs populations respectives étant dans un rapport considérable de l'ordre de 1 à 1 million voire plus. L'invention a donc pour objet ce produit nouveau obtenu par l'interruption d'une décomposition enzymatique particulière partielle selon le procédé précédemment défini d'un produit amylacé; il comporte après purification au moins de l'amidon partiellement dégradé et au moins quelques levures.

Le produit à l'état brut est caractérisé par le fait que la teneur en amidon partiellement dégradé est comprise entre environ 50 et 98% (dans le cas d'un substrat amylacé épuré), le reste des composants étant représenté par les constituants insolubles du substrat (fibres, protéines, lipides, cendres, minéraux, ions), les levures les enzymes, et quelques bactéries et son pH étant compris entre 4 et 8 selon l'enzyme ou les enzymes utilisées.

L'invention porte encore sur les applications du produit, notamment:
- application comme agent liant; comme agent de charge; comme agent gélifiant;
- application comme support d'arômes;
- application comme additif d'extrusion;
- application comme agent diététique, par exemple comme sucre lent; et comme agent d'alimentation infantile sans gliadine;
- application du produit stabilisé, et conservé par ensilage pour la production de produits d'alimentation animale.

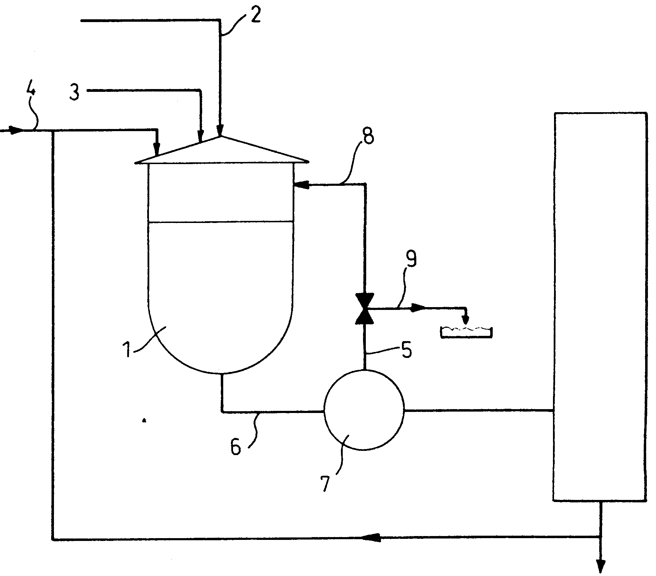

Afin de mieux comprendre l'invention on a illustré sur la figure unique du dessin annexé un schéma de l'installation pour la mise en oeuvre de ce procédé.

Le produit, encore appelé "sédiment" est extrait d'une installation de transformation par hydrolyse et fermentation simultanées.

De manière connue, l'installation comporte un récipient 1 ou fermenteur. Celui-ci est alimenté en continu en produit amylacé brut 2 finement broyé (farine), en enzymes 3, en eau 4, et en sédiments recyclés 5. Le produit est brut en ce sens qu'il ne subit de préférence aucune épuration ou autre traitement particulier. Le fermenteur comporte de plus une sonde de températures reliée à un dispositif de régulation de température non représenté, et une sonde de pH reliée à une alimentation en soude non représentée également.

Le fermenteur comporte une sortie d'évacuation 6 en continu des vins qui alimente un décanteur horizontal centrifuge 7.

Ce décanteur est un séparateur, mais par rapport aux autres types de séparateurs (filtres à plaques, tambour rotatif ou sous pression, décanteur hydrocyclone, ultrafiltration, séparateur centrifuge à assiettes ou à buses) il présente l'avantage de séparer de manière simple et sûre les matières insolubles de l'effluent chargé d'alcool.

On obtient par exemple en sortie de décantation un flux de sédiments et d'effluents respectivement de 40% et 60% en masse et la matière sèche du sédiment est comprise entre 42 et 48% (P/P).

Le sédiment est en partie extrait et en partie recyclé dans le récipient de manière à éviter l'épuisement de la biomasse dans le récipient notamment des levures de fermentation.

La proportion de recyclage dépend essentiellement des quantités relatives d'éthanol et/ou de sédiment que l'on veut produire. Elle varie entre 90% (le sédiment selon l'invention ne représente que 10% ) et 50% de la masse totale de sédiments en sortie du décanteur.

On peut même prévoir un prélèvement de la totalité, c'est à dire l'absence de recyclage.

A titre d'exemple on a réalisé l'essai suivant: pour un récipient de volume utile 100 litres, en fonctionnement continu avec recyclage, pour un temps de séjour dans le récipient de l'ordre de 33 heures, à pH 4 et à la température de 33°C:
- débit de farine: 0,9 kg/h
- enzymes: solution AMG à 5,8.10-3 (litre d'enzyme/litre de vin).
- débit d'eau: 2,1 kg/h
- débit de sortie vers décanteur: 4,75 kg/h
- débit d'effluents (vins): 2,75 kg/h
- débit de produits décantés (drêches, sédiment et autres):2 kg/h
- extrait: 25% soit 0,50 kg/h
- débit de produits recyclés (75%): 1,50 kg/h

On a réalisé à une échelle plus importante de plusieurs dizaines de tonnes par jour, la transformation du blé en éthanol selon ce procédé, avec les mêmes enzymes et en mêmes proportions. On obtient les quantités suivantes:
- I -: 60 tonnes/jour de farine
200 hectolitres d'éthanol
6,6 tonnes de sédiment selon l'invention
9 tonnes de son
9,5 tonnes de drêches
- II -: 100 tonnes/ jour de farine
200 hectolitres d'éthanol
9,5 tonnes de sédiment selon l'invention
15 tonnes de son
15,8 tonnes de drêches

On peut donc, comme on le voit, faire varier la production d'amidon partiellement hydrolysé selon l'invention c'est à dire de sédiment, pour une même production d'éthanol. Inversement il est possible, pour un débit de farine constant, de moduler la quantité d'extrait sédimentaire par rapport à celle d'éthanol.

Le produit obtenu selon ce procédé se présente après dessiccation par lyophilisation sous la forme de poudre ou après séchage à chaud sous la forme de flocons; sa composition pour un temps de séjour dans le récipient d'hydrolyse d'environ 40 heures, pour l'exemple de réalisation précédent est environ la suivante:
- - amidon :: 72% ± 2%
- - fibres :: 4,8% ±2%
- - protéines :: 7%
- - lipides :: 0,5% ±0,3%
- - cendres :: 0,9% ±0,5%
- - minéraux en ppm:: Cu: 7,3, Zn: 47, Fe: 124, Mn:12,5
- - ions:: NH4: 0,25%, PO4: 0,34%, Cl2: 372 ppm, NO2 < 0,01%, NO3 < 0,01%

et la partie amylacée ne présente pas de dextrines résiduelles entre G2 et G10;
Le pH du produit est situé environ entre 3,8 et 4, sa densité est de l'ordre de : d réelle = 0,96 et d apparente = 0, 214.

Bien entendu cette composition est éminemment variable et dépend en grande partie de la qualité du substrat employé, ici de la farine de blé, mais aussi du choix et de la concentration d'enzymes, du temps de séjour, etc.

Le produit selon l'invention est remarquable par ses nombreuses applications et utilisations dans le secteur des produits alimentaires : en charcuterie, dans l'élaboration des plats cuisinés, des sauces, des entremets et desserts lactés, les boissons, la biscuiterie et la panification, le Peet-Food, etc.

En outre le procédé selon l'invention et son produit présente l'avantage particulier d'être exempt de gliadine même lorsque que le substrat employé est du blé ce qui permet de l'employer en alimentation infantile.

La gliadine est connue pour être un produit impropre à la consommation infantile. Or la farine de blé, couramment utilisée pour la production d'éthanol comporte, entre autres produits, de la gliadine. Le procédé selon l'invention est particulièrement intéressant par le fait que le sédiment est pratiquement exempt de gliadine: celle-ci, solubilisée dans le fermenteur, est séparée du sédiment pendant la décantation et se retrouve dans les effluents.

Les mesures ont montré que au moins 95% de la gliadine est éliminée dans les effluents, les au plus 5% restants se retrouvent dans la fraction contenant le sédiment. La très faible quantité de gliadine extraite avec le sédiment peut alors sans difficulté être éliminée car elle est soluble dans l'alcool.

De nombreuses applications de ce produit sont possibles, soit directement avec le produit extrait, soit après traitements secondaires à l'issue de l'extraction.

En effet la présentation et les applications du produit varient selon l'aspect et/ou la manière dont a été arrêtée l'hydrolyse.

Avant stabilisation le sédiment peut subir un traitement préalable destiné à éliminer quelques substances indésirables: on le dilue en solution aqueuse en présence d'alcool ou de sel ou de produits modifiant le pH. La solution d'alcool, par exemple élimine les traces de gliadine.

Le sédiment est stabilisé soit par:
- séchage à froid: par lyophilisation, ce qui donne une poudre,
- séchage à chaud sur bande ou sur cylindre: le sédiment gonfle et donne un film transformé en flocons,
- atomisation : le sédiment est pulvérisé en particules dans une tour d'atomisation connue en soi, les particules étant séchées dans un courant d'air chaud,
- ensilage avec introduction de bactéries appropriées connues en soi.

D'autre part le sédiment est purifié par séparation mécanique de type connu en soi, la purification pouvant aller jusqu'à ne conserver essentiellement que les grains d'amidon partiellement hydrolysés.

Une première application est la mise en oeuvre de sa fonction nutritionnelle: par exemple en diététique le sédiment a une action sur le transit intestinal.

Une seconde application est la mise en oeuvre de ses fonctions techniques : il entre dans la composition de plats cuisinés soit:
- comme agent liant, en raison de ses capacités à la fois émulsifiante, épaississante et gélifiante. On peut en particulier l'utiliser dans l'alimentation des nourrissons, à de très faibles doses, pour combattre les spasmes de l'estomac entraînant des vomissements;
- comme agent émulsifiant: par exemple il entre dans la composition d'émulsions que l'on cherche à stabiliser dans le temps, sans pour autant atteindre le stade de la gélification (mayonnaise industrielle, par exemple);
- comme agent épaississant: par exemple pour le maintien de la consistance des sauces dans les plats cuisinés prêts à la consommation et conservés au froid positif;
- comme agent gélifiant: par exemple, dans des préparations culinaires ou aliments infantiles devant être maintenus au froid positif: le sédiment se gélifie en moins de 16 heures à 4°C dans l'eau froide et en quelques minutes à température ambiante et se maintient à l'état de gel en le chauffant;
- comme agent d'extrusion: par exemple en biscuiterie, où l'on observe en particulier un gain sur l'énergie dépensée pour l'extrusion de l'ordre de 20%. Dans ce cas d'application le produit peut être utilisé à l'état brut, sans traitement secondaire de purification;
- comme agent de charge: par exemple en biscuiterie, pâtisserie, confiserie grâce à son pouvoir élevé de rétention d'arômes, d'eau et de matières grasses. Dans ce type d'application le produit subit de préférence, après extraction un ou plusieurs traitements d'épuration destinés à éliminer le ou les constituants non désirés: fibres, levures, etc;
- comme agent diététique: par exemple pour lutter contre le diabète juvénile insulino-dépendant chez l'homme, grâce à un effet retard, observé sur des rats diabétiques; le sédiment se comporte en effet comme un "sucre lent". Cet effet "sucre lent" est également avantageux en aliment infantile car il régularise la digestion du nourrisson.

Une troisième catégorie d'application est l'utilisation en alimentation animale herbivore: le produit transformé et conservé par ensilage peut être très avantageusement donné en nourriture au bétail producteur de lait, notamment aux vaches laitières. La production de lait s'en trouve sensiblement augmentée: on a observé des augmentations de production de 15 à 20%.

Le produit obtenu est particulièrement avantageux du fait que, après extraction et stabilisation il peut être conservé sans traitement particulier et utilisé directement sans traitement préalable. Aucune prolifération bactérienne ne se produit du fait notamment de la présence des levures.

Le procédé permet donc d'obtenir un produit stable, c'est à dire de composition constante pour un substrat déterminé, et peu coûteux eu égard à la faible consommation d'enzyme et d'énergie.

## Revendications

**1** **-** Procédé de production en continu d'amidon partiellement hydrolysé, par hydrolyse à froid et fermentation simultanée d'un substrat amylacé brut broyé, dans lequel on réalise de manière connue en soi une hydrolyse du substrat amylacé avec une préparation enzymatique libérant des sucres, et dans laquelle on ajoute des levures, caractérisé en ce que:
- on soutire le jus obtenu après hydrolyse pendant une durée allant de 20 à 60 heures, lequel contient les produits solubilisés, et des produits non solubilisés,
- de ce jus on sépare tout ce qui n'est pas solubilisé par l'hydrolyse, que l'on recycle,
- avant recyclage on extrait en tout ou partie cette fraction non solubilisée contenant l'amidon partiellement hydrolysé et on la stabilise.

**2** **-** Procédé selon la revendication 1 caractérisé en ce que la stabilisation est réalisée par séchage, congélation, atomisation ou ensilage.

**3** **-** Produit amylacé partiellement hydrolysé résultant du procédé selon l'une quelconque des revendications précédentes.

**4** **-** Produit selon la revendication 3 caractérisé par le fait qu'il comporte au moins de l'amidon partiellement dégradé et au moins quelques levures.

**5** **-** Produit selon la revendication 4 caractérisé par le fait que la teneur en amidon partiellement dégradé est comprise entre 50 et 98 %, le reste des composants étant représenté par les constituants non solubles du substrat (fibres, protéines, lipides, cendres, minéraux, ions), des levures, les enzymes et quelques bactéries, et son pH est compris entre 4 et 8.

**6** **-** Application du produit selon l'une quelconque des revendications 3, 4, 5, dans l'industrie alimentaire comme support d'arômes.

**7** **-** Application du produit selon l'une quelconque des revendications 3, 4, 5 dans l'industrie alimentaire comme additif d'extrusion.

**8** **-** Application du produit selon l'une quelconque des revendications 3, 4, 5 dans l'industrie alimentaire comme agent diététique.

**9** **-** Application du produit amylacé partiellement hydrolysé résultant du procédé selon la revendication 1, stabilisé, et conservé par ensilage, dans la production de produits d'alimentation animale.
